# EUROPEAN PATENT SPECIFICATION

(11) **EP 1 011 743 B1**
(45) Date of publication and mention of the grant of the patent: **27.07.2011**
(21) Application number: 98939379.8
(22) Date of filing: 13.08.1998
(51) Int. Cl.: A61L 27/00, A61L 29/00, A61L 31/00

(54) **LOADING AND RELEASE OF WATER-INSOLUBLE DRUGS**
BELADUNG UND ABGABE VON WASSERUNLÖSLICHEN WIRKSTOFFEN
MISE EN CHARGE ET LIBERATION DE MEDICAMENTS INSOLUBLES DANS L'EAU

(30) Priority: 13.08.1997 US 910136
(43) Date of publication of application: 28.06.2000
(73) Proprietor: Boston Scientific Limited, St. Michael, Barbados, West Indies (BB)
(72) Inventor: BARRY, James, Marlboro, MA 01752 (US); PALASIS, Maria, Wellesley, MA 02181 (US)
(74) Representative: Peterreins, Frank
(86) International application number: PCT/US1998/016775
(87) International publication number: WO 1999/008729

(56) References cited:
- EP-A- 0 623 354
- WO-A-92/11896
- WO-A-92/15286
- WO-A-93/06792
- WO-A-94/21308
- WO-A-95/03036
- WO-A-95/08305
- WO-A-95/21636
- WO-A-96/25176
- WO-A-96/32907
- WO-A-97/10011
- WO-A-97/25085
- US-A- 5 439 446
- US-A- 5 624 411
- US-A- 5 651 986

## Description

### FIELD OF THE INVENTION

The invention relates to devices for the localized delivery of substantially water-insoluble drug agents within the body, and methods for preparing such devices.

### BACKGROUND

The systemic administration of drug agents, such as by transoral or intravenous means, treats the body as a whole even though the disease to be treated may be localized. In such a case, systemic administration may not be desirable because, for example, the drug agents may have unwanted effects on parts of the body which are not to be treated, or because treatment of the diseased part of the body requires a high concentration of drug agent that may not be achievable by systemic administration.

It is therefore often desirable to administer drug agents at a localized site within the body. Common examples include cases of localized disease or occluded body lumens. Various methods have been proposed for such localized drug administration. For example, U.S. Patent No. 5, 304, 121, discloses a method of delivering water-soluble drugs to tissue at desired locations of a body lumen wall. The method generally includes the steps of impregnating a hydrogel polymer on an expandable catheter with an aqueous drug solution, inserting the catheter into a blood vessel to a desired location, and expanding the catheter against the surrounding tissue allowing the release of the drug to the tissue. This method of localized drug delivery using hydrogel polymer impregnation has a limitation of being applicable to drug agents which are dissolved in water at concentrations sufficient for therapeutic gel loading levels. There thus exists a need for a method and apparatus for the localized delivery of drug agents within the body, where the drug agents are substantially water-insoluble.

### SUMMARY OF THE INVENTION

One objective of the present invention is to provide a apparatus for the localized delivery of substantially water-insoluble drug agents to predetermined locations within the human body, and methods for preparing the device.

The present invention provides medical devices for the localized delivery of substantially water-insoluble drugs agents, and methods for preparing such medical devices.

The present invention features a balloon catheter for delivering substantially water-insoluble drug agents to tissue at a desired location along body lumen walls. The balloon catheter is constructed for insertion in a body lumen and has a catheter shaft and an expandable portion mounted on the catheter shaft. The expandable portion is expandable to fill the cross-section of the body lumen. At least a portion of the exterior surface of the expandable portion is defined by a polymer coating. Incorporated into the polymer coating is at least one substantially water-insoluble drug. The catheter is positioned to a desired target location within the body, whereupon the polymer coating absorbs water, thus dissolving the drug and resulting in the diffusion of the drug out of the polymer coating. The polymer and drug are selected to allow controlled release of a desired dosage of the drug from the polymer.

### BRIEF DESCRIPTION OF THE DRAWINGS

Fig. 1a shows one embodiment of the present invention in which a drug solution is impregnated into a polymer-coated balloon catheter.
Fig. 1b shows the insertion of a polymer-coated balloon catheter into a body lumen, in accordance with the present invention.
Fig. 1c shows the expansion of a polymer-coated balloon catheter at an occlusion site within a body lumen, in accordance with the present invention.
Fig. 2 shows a drug delivery balloon catheter embodiment of the present invention including a sheath for covering the catheter as it is being moved through a vessel toward the occlusion to be treated.
Figs. 3a and 3b show the release profile of paclitaxel from a balloon catheter having a polyacrylic acid-based coating for up to 50 and 5000 minutes, respectively, in accordance with the present invention.
Figs. 4a and 4b show the release profile of dexamethasone from a balloon catheter having a polyacrylic acid-based coating for up to 30 and 400 minutes, respectively, in accordance with the present invention.
Fig. 5 shows the release profiles of molsidomine from various balloon catheters having a polyacrylic acid-based coating for up to 5 minutes, in accordance with the present invention.
Figs. 6a and 6b show the release profiles of water-soluble and substantially water-insoluble estradiol from balloon catheters having a polyacrylic acid-based coatings for up to 10 and 200 minutes, respectively, in accordance with the present invention.
Fig. 7 shows the release profile of paclitaxel for up to 10 days from polyurethane coated stents dipped in 30 mg/ml paclitaxel in ethanol for 3 days, in accordance with the present invention.
Fig. 8 shows the release profiles of paclitaxel from various polyurethane-coated balloon catheters for up to 2 hours, in accordance with the present invention.

### DETAILED DESCRIPTION

The present invention provides medical devices for the localized delivery of one or more substantially water-insoluble drug agents to predetermined locations within the human body, and methods for preparing such medical device.

In accordance with an embodiment of the invention, a substantially water-insoluble drug agent is dissolved in a volatile organic solvent. "Organic solvent" is intended to mean a singular organic solvent or a solvent mixture having at least one organic component. The solvent mixture also includes mixtures of water with miscible organic solvents. The drug solution is then applied to a polymer coating on a medical device that is adapted for insertion into the body. The medical device is a balloon.

In the invention, the polymer is provided in the form of a coating on an expandable portion of a balloon catheter. After applying the drug solution to the polymer and evaporating the volatile solvent from the polymer, the balloon catheter is inserted into a body lumen where it is positioned to a target location. The expandable portion of the balloon catheter is subsequently expanded to bring the drug-impregnated polymer coating into contact with the lumen wall. The drug is released from the polymer as it slowly dissolves into the aqueous bodily fluids and diffuses out of the polymer. This enables administration of the drug to be site-specific, limiting the exposure of the rest of the body to the drug.

The polymer used in the present invention is preferably capable of absorbing a substantial amount of drug solution. When applied as a coating on a medical device in accordance with the present invention, the dry polymer is typically on the order of about 1 to 10 microns thick, preferably about 2 to 5 microns. Very thin polymer coatings, e.g., of about 0.2-0.3 microns and much thicker coatings, e.g., more than 10 microns, are also possible. It is also within the scope of the present invention to apply multiple layers of polymer coating onto a medical device. Such multiple layers are of the same or different polymer materials.

The polymer of the present invention is hydrophilic or hydrophobic, and is selected from the group consisting of polycarboxylic acids, cellulosic polymers, gelatin, polyvinylpyrrolidone, maleic anhydride polymers, polyamides, polyvinyl alcohols, polyethylene oxides, glycosaminoglycans, polysaccharides, polyesters, polyacrylamides, polyethers, and copolymers thereof. Coatings from polymer dispersions such as acrylic latex dispersions are also within the scope of the present invention. The preferred polymer is polyacrylic acid, available as HYDROPLUS (Boston Scientific Corporation, Natick, Mass.), and described in U.S. Pat. No. 5,091,205 U.S. Patent No. 5,091,205 describes medical devices coated with one or more polyisocyanates such that the devices become instantly lubricious when exposed to body fluids.

By "substantially water-insoluble drug" is meant any therapeutic agent having a greater solubility in organics than in water. More specifically, such drugs have a water solubility of no greater than 1 part drug to . 30 parts water, more typically no greater than 1 part drug to 1,000 parts water. Such solubilities are described as "sparingly soluble" to "very slightly soluble" in the art.

The drug agents used in the present invention are selected from a number of drug types depending on the desired application. For example, these drugs include anti-inflammatory agents such as dexamethasone, prednisolone, corticosterone, budesonide, estrogen, sulfasalazine, mesalamine, and analogues thereof; antineoplastic/ antiproliferative/anti-miotic agents such as paclitaxel, 5-fluorouracil, cisplatin, vinblastine, vincristine, epothilones, endostatin, angiostatin, thymidine kinase inhibitors, and analogues thereof; anesthetic agents such as lidocaine, bupivacaine, ropivacaine, and analogues thereof; anti-coagulants; and growth factors.

In accordance with the present invention, the drug agents are dissolved in a volatile organic solvent such as, for example, ethanol, isopropanol, chloroform, acetone, pentane, hexane, or methylene chloride, to produce a drug solution. The drug solution is then applied to the polymer. A volatile organic solvent typically is selected to provide drug solubilities much greater than the corresponding aqueous solubility for the substantially water-insoluble drug. Accordingly, application of the drug solution to the polymer often results in drug loadings that are orders of magnitude greater than loadings that can be achieved by application of a saturated aqueous solution of the drug to the polymer.

The drug solution is applied to the polymer coating by any suitable means, including dipping the polymer coating into the drug solution or by applying the solution onto the coating such as by pipet. In the former method, the amount of drug loading is controlled by regulating the time the polymer is immersed in the drug solution, the extent of polymer cross-linking, the concentration of the drug in the solution and/or the amount of polymer coating.

After applying the drug solution to the polymer coating, the volatile solvent is evaporated from the coating, for example, by drying in air or in an oven.

The release profile of the drug from the polymer coating is determined by many factors including the drug solubility, and the thickness and porosity of the polymer coating. When an expandable member such as a balloon catheter is used to administer the drug, pressure can be used to increase the rate of drug transfer to the tissue. An increase in pressure increases the diameter of the balloon and therefore the diameter of the surrounding tissue, thereby increasing the surface area for drug transfer. The amount of drug that is delivered per unit time is therefore increased.

The balloon catheter of the present invention also optionally comprises a sheath member which is extendable over the expandable portion to inhibit release of the drug into body fluids during placement of the catheter.

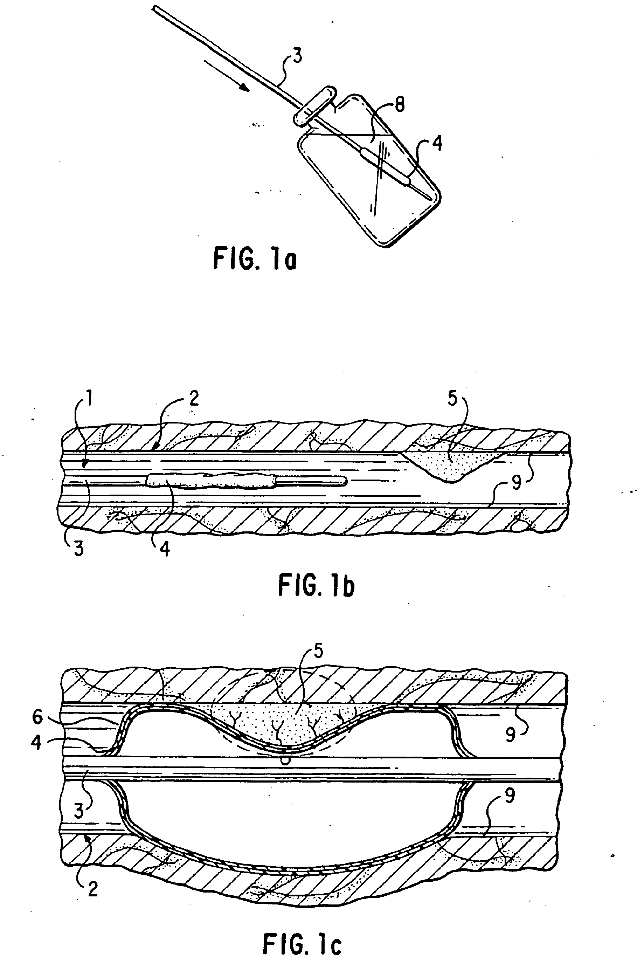

Referring now to Figs. 1a-1c, an embodiment for the localized delivery of substantially water-insoluble drugs to a predetermined location within the body is described. The drug administration method shown in Figs. la-lc illustrates the use of the present invention in conjunction with an angioplasty process. Catheter device 1 comprises a body 3 having a balloon 4 attached at its distal end. The balloon 4 on the catheter 3 includes a polymer coating 6. As shown in Fig. 1a, drug solution 8 is impregnated into the polymer coating with the balloon in its substantially deflated state prior to insertion into the patient. As shown in Fig. 1b, after the volatile solvent is evaporated, the device 1 is inserted into a body lumen 2 having a region to be treated, such as an occlusion due to a deposition of plaque 5 on the lumen wall tissue 9. The device 1 is moved along the vessel to position the balloon 4 at the occlusion site, as shown in Fig. lc. The lumen may be, for example, a narrow, tortuous opening through which the catheter is passed by torquing or other known techniques. As shown in Fig. 1c, the balloon is inflated to provide close contact between the drug-impregnated polymer coating 6 and the surrounding plaque and tissue. As water from the body penetrates into the polymer coating 6, it begins to dissolve the drug agent, which subsequently diffuses out of the polymer coating 6 and into the surrounding plaque and tissue.

During drug administration, a substantial amount of the drug contained in the polymer coating is diffused into the affected area. The inflation pressure needed to expand the balloon catheter and dilate the lumen, if necessary, is typically in the range of about 1 to 20 atm. The balloon is formed of any suitable materials such as vinyl polymers such as polyethylene; polyesters such as polyethylene terephthalate; polyamides such as nylon; polyolefins and copolymers thereof (e.g., Selar, Pebax, Surlyn, Hytrel, etc.). The balloon is optionally a perfusion balloon, which allows blood to perfuse the catheter to prevent ischemia during delivery. A perfusion balloon is particularly preferred for long arterial delivery times and when the delivery drug is only very slightly soluble in water.

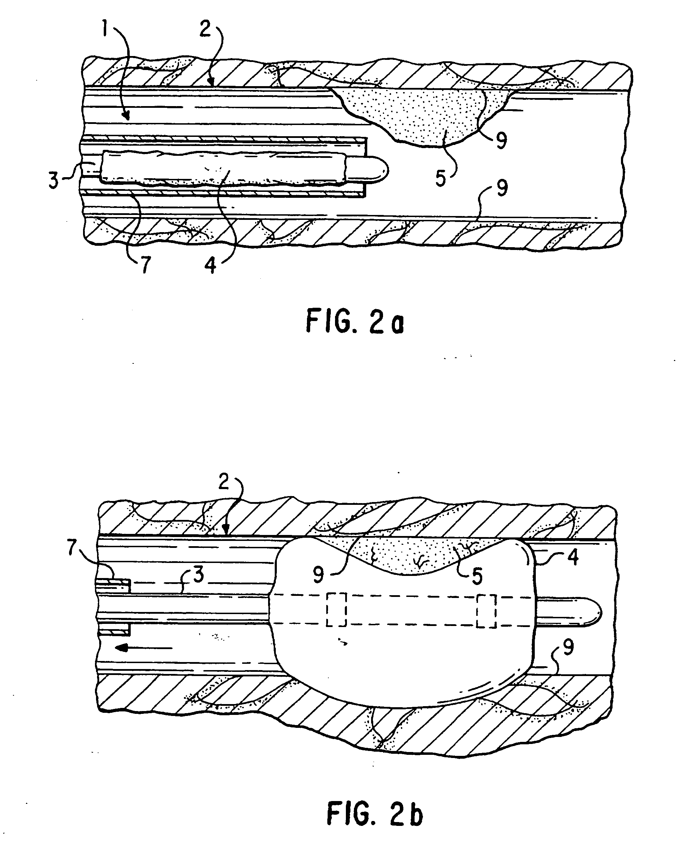

Referring to the embodiment of the invention illustrated in Fig. 2, the balloon portion 4 of catheter 3 is optionally covered by a protective sheath 7 while the instrument 1 is inserted into a body lumen 2 and positioned at a treatment region. As the coated balloon 4 is positioned at occluded site 5, the protective sheath 7 is drawn back to expose the balloon 4. In an alternative embodiment, the sheath remains stationary while the catheter moves the coated balloon forward into the occluded region. The sheath 7 protects the coating and inhibits premature release of the drug. Such a sheath might be particularly advantageous when using drugs which are not sufficiently water-insoluble or if even minor delivery to tissue during catheter placement is a problem, e.g. for extremely toxic drugs.

Although Figs. 1 and 2 illustrate the application of the present invention to an angioplasty process, the present invention is also used to administer drug agents to target locations where there is no occlusive formation.

Procedures for preparing a drug delivery balloon catheter with a polymer coating are presented in the following non-limiting examples.

### Example 1: Release kinetics of paclitaxel from polyacrylic acid-based coating.

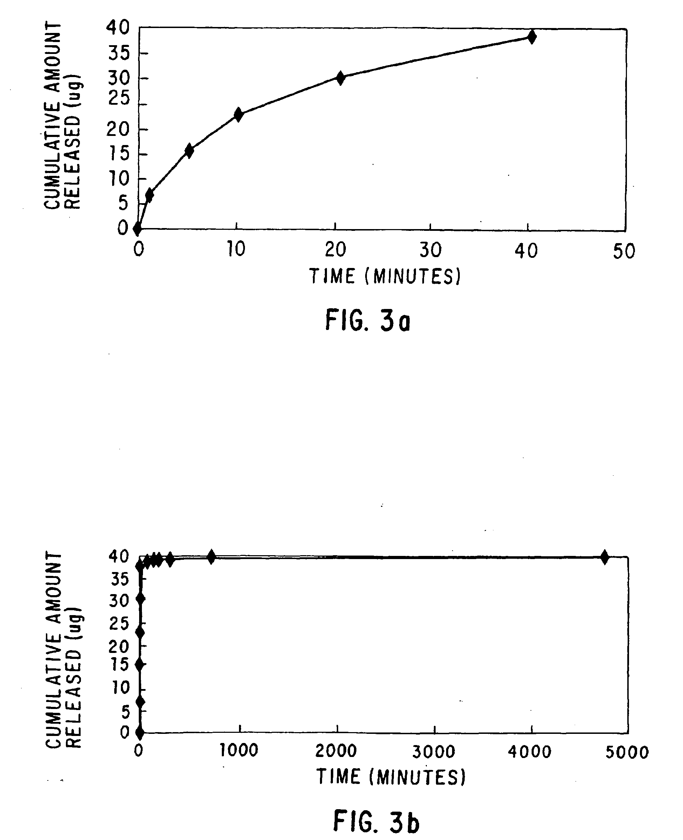

A 2 mg/ml solution of paclitaxel is prepared in chloroform. The solution is gently agitated until the paclitaxel is completely dissolved. The solution is applied via pipet to a balloon catheter having a polyacrylic acid-based coating and inflated to 2 atm. A total of 100 µl of solution, and hence 200 µg of paclitaxel, is applied to the catheter. The balloon catheter is then dried in air for 30 minutes and in a vacuum oven for 48 hours at 50°C to evaporate the chloroform. The catheter is then immersed in a solution of 1% dimethyl sulfoxide (DMSO) and phosphate buffered saline (PBS) having a pH of 7.4 for in-vitro drug release. The cumulative amount of paclitaxel released from the catheter coating yields the data shown in Figs. 3a and 3b.

### Example 2: Release kinetics of dexamethasone from polyacrylic acid-based coating.

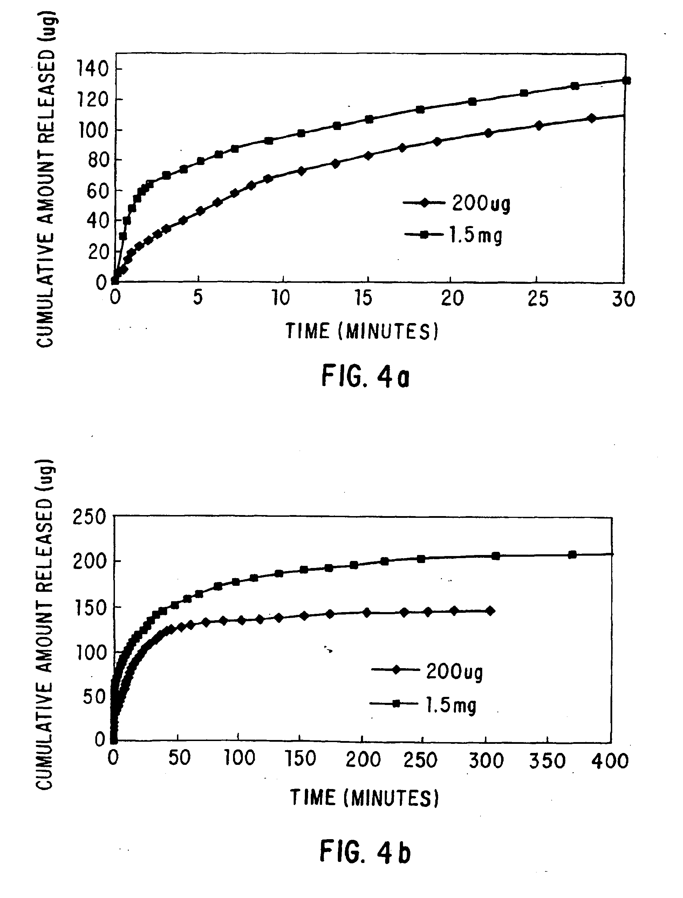

Solutions containing 1.5 mg/ml and 200 µg/ml of dexamethasone in chloroform, are prepared by gently agitating until the dexamethasone is completely dissolved. The solutions are separately applied via dripping to separate balloon catheters having polyacrylic acid-based coatings and inflated to 2 atm. A total of 100 µl of each solution is applied to each respective catheter, corresponding to dexamethasone loadings of 150 µg and 20 µg, respectively. These results can be contrasted with the inability to apply substantial amounts of dexamethasone to polyacrylic acid-based coatings using aqueous solutions, in which case only about 1 µg of dexamethasone can be loaded into such coatings. The balloon catheters are then dried in a vacuum oven for 2 hours at 50°C to evaporate the chloroform solvent. The catheters are thereafter immersed in PBS (pH = 7.4) to track the release of dexamethasone over time. The cumulative amount of dexamethasone released from each catheter yields the data shown in Figs. 4a and 4b.

### Example 3: Release kinetics of molsidomine from polyacrylic acid-based coating.

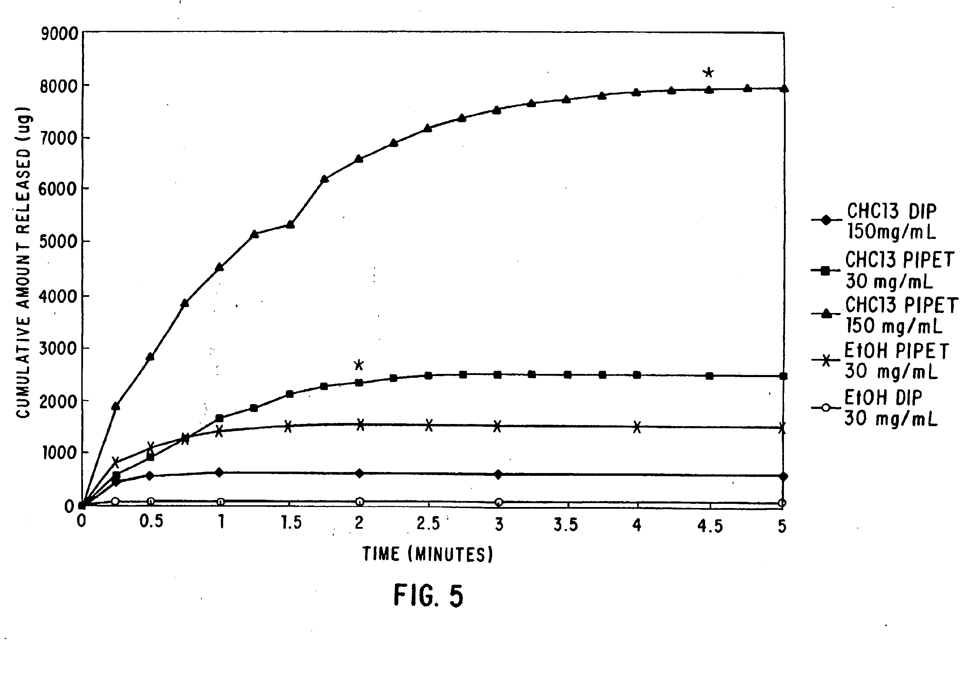

Various solutions of molsidomine in volatile solvents are prepared and applied to balloon catheters by the methods indicated in Table I. In the "dip" application technique, each balloon catheter having a polyacrylic acid-based coating is dipped into its respective solution for 10 minutes. In the "pipet" application technique, 200 µl of solution is pipetted onto its respective coated balloon catheter while slowly turning. All samples are dried in an oven for 30 minutes at 50°C and thereafter immersed in PBS (pH = 7.4) to track the release of molsidomine over time. The cumulative amount of molsidomine released from each catheter yields the data shown in Fig. 5a and 5b.

**Table I. Molsidomine solution characterization, and methods of applying molsidomine solution to polymer coated catheters.**

| Sample | solvent | Concentration (mg Molsidomine per ml solvent) | Application technique |
|---|---|---|---|
| 1 | chloroform | 150 | dip |
| 2 | chloroform | 30 | pipet |
| 3 | chloroform | 150 | pipet |
| 4 | ethanol | 30 | pipet |
| 5 | ethanol | 30 | dip |

### Example 4: Comparative release kinetics for water-soluble and water-insoluble estradiol.

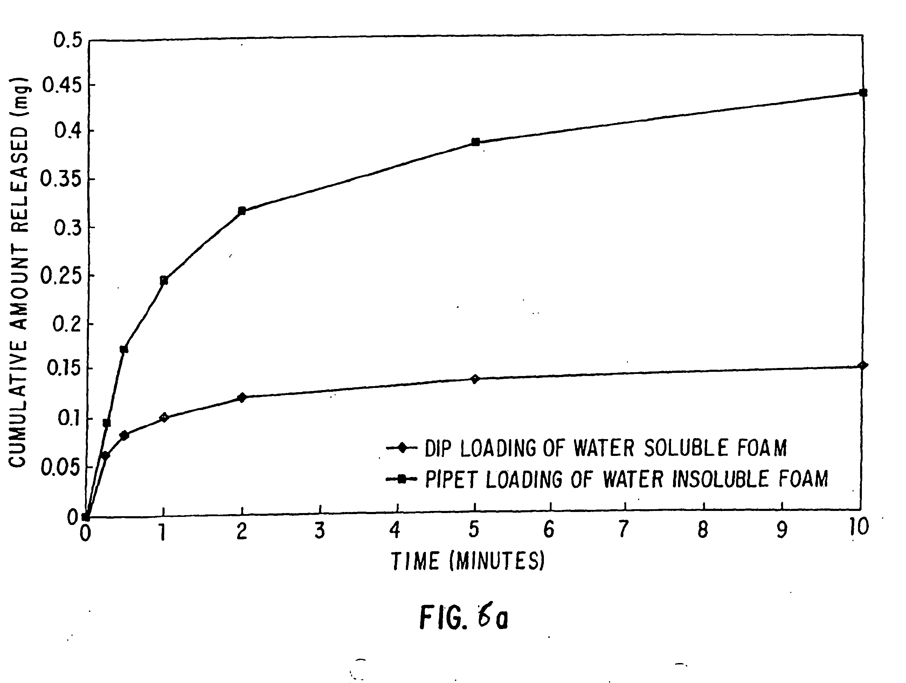

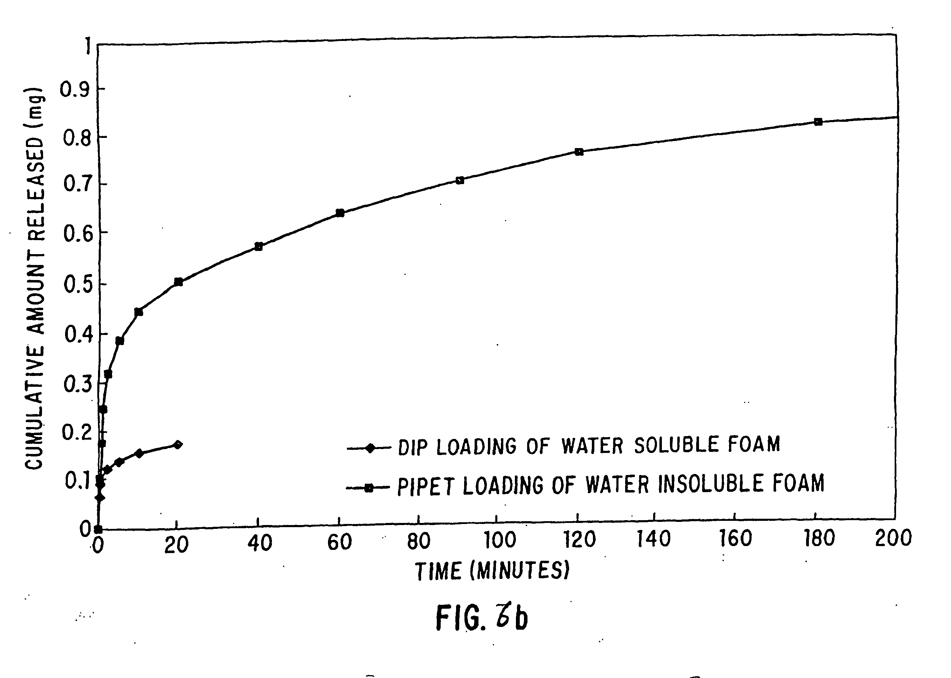

Estradiol is provided in both water-soluble and substantially water-insoluble forms. Water-soluble estradiol is applied to a balloon catheter coated with a polyacrylic acid-based coating by i) preparing a 10 mg/ml solution of water-soluble estradiol in deionized, ultra-filtered water; and ii) placing the balloon catheter, inflated to 2 atm, into 200 µl of the solution for 20 minutes. Water-insoluble estradiol is applied to a balloon catheter coated with a polyacrylic-acid based coating by i) preparing a 10 mg/ml solution of substantially water-insoluble estradiol in methanol; and ii) dripping 100 µl of the solution onto the balloon catheter. The catheters are thereafter immersed in PBS (pH = 7.4) to track the release of both water-soluble and water-insoluble estradiol over time. Greater release is observed for the substantially water-insoluble form of estradiol when compared to the water-soluble form. The cumulative amount of estradiol released from each catheter yields the data shown in Figs. 6a and 6b.

### Example 5: In-vivo delivery of paclitaxel from polyacrylic acid-based coating.

A 9.8 mg/ml solution of radio-labeled paclitaxel in chloroform is prepared. A total of 50 µl of the solution is applied via pipet to a balloon catheter having a polyacrylic acid-based coating. The paclitaxel from the coated balloon catheter is then released in-vivo to porcine arteries. After release for a predetermined amount of time, the paclitaxel remaining in the coating is extracted using two sequential ethanol washes. The amount of paclitaxel released in the pig bloodstream, as calculated from the amount of paclitaxel loaded into the coating minus that extracted from the coating after delivery, is shown in Table II.

**Table II. Amount of paclitaxel released into pig bloodstream from an impregnated, polyacrylic acid-based coated balloon catheter, as a function of delivery time.**

| Amount of time in bloodstream | Amount of paclitaxel extracted from balloon after delivery (µg) | Amount of paclitaxel released in bloodstream (µg) | % of paclitaxel released in bloodstream |
|---|---|---|---|
| 1 minute | 182 ± 1 | 307 | 63 |
| 5 minutes | 160 ± 30 | 330 | 68 |

### Example 6: Delivery of paclitaxel to explanted porcine arteries from polyacrylic acid-based coating.

A 9.8 mg/ml solution of radio-labeled paclitaxel in chloroform is prepared. A total of 50 µl of the solution is applied via pipet to a balloon catheter having a polyacrylic acid-based coating. The coated balloon catheter is then delivered to an explanted porcine artery for 15 minutes. After delivery, the paclitaxel remaining in the coating is extracted using two sequential ethanol washes. The delivered paclitaxel is extracted from the vessel, also by using two sequential ethanol washes. In addition, the vessel.is placed in tissue solvent and counted for paclitaxel. Using these extraction methods, at least 80% of the paclitaxel loaded onto the balloon catheter is recovered, as shown in Table III.

**Table III. Paclitaxel recovery from ex vivo delivery to porcine artery.**

| | |
|---|---|
| Amount paclitaxel loaded onto balloon | 489 µg |
| Amount paclitaxel extracted from the balloon after delivery | 360 µg |
| Amount paclitaxel extracted from artery | 30 µg |
| Amount paclitaxel counted from tissue solution | 1 µg |
| Total paclitaxel measured | 391 µg |
| Percentage of paclitaxel recovered | 80% |

### Reference Example 7: Release kinetics of paclitaxel from polyurethane-based stent coating.

slotted tube stainless steel stents are coated with polyurethane by spraying a 1 wt% solution of CHRONOFLEX polyurethane (made by CT Biomaterials) in tetrahydrofuran directly onto the stent surface. The coated stents are dried in a vacuum oven for three hours at 70°C.

Each polyurethane coated stent is placed in a vial, which is filled to maximum volume (1.5 ml) with a solution of paclitaxel in ethanol, and sealed. The stent is stored in the vial for three days at room temperature. The stent is then removed from the vial and dried for one hour at 65°C.

The above procedure is conducted using solutions of varying concentrations. Each stent is analyzed for paclitaxel content by extraction in dichloromethane solvent. The results are presented in Table IV below. Samples 1 and 2 were obtained using a paclitaxel concentration of 10 mg/ml, samples 3 and 4 using a 20 mg/ml solution and sample 5 and 6 using a 30 mg/ml solution.

**Table IV. Paclitaxel content.**

| Sample # | Paclitaxel conc. (mg/ml) | Paclitaxel content (µg) | Coating. Wt. (µg) | µg Paclitaxel per µg coating |
|---|---|---|---|---|
| 1 | 10 | 44.8 | 796 | 0.06 |
| 2 | 10 | 88.2 | 859 | 0.10 |
| 3 | 20 | 151.2 | 718 | 0.21 |
| 4 | 20 | 127.6 | 702 | 0.18 |
| 5 | 30 | 157.1 | 736 | 0.21 |
| 6 | 30 | 144.3 | 629 | 0.23 |

These results suggest that Paclitaxel loading is relatively independent of paclitaxel concentration above 20 mg/ml, assuming equilibrium is attained in the three-day period. Nevertheless, the 30 mg/ml paclitaxel concentration is chosen for release studies as it produces the maximum paclitaxel loading (21-23%), while still being sufficiently below the saturation concentration for paclitaxel in ethanol (39 mg/ml).

Seven polyurethane coated stents are loaded using a 30 mg/ml paclitaxel solution, removed and dried as set forth above. Paclitaxel from four of the stents is extracted in dichloromethane solvent. The results of this extraction are presented in Table V below:

**Table V. Paclitaxel content.**

| Sample # | Paclitaxel conc. (mg/ml) | Paclitaxel content (µg) | Coating Wt. (µg) | µg Paclitaxel per µg coating |
|---|---|---|---|---|
| 1 | 30 | 111.7 | 676 | 0.17 |
| 2 | 30 | 50 | 627 | 0.08 |
| 3 | 30 | 45.3 | 612 | 0.07 |
| 4 | 30 | 37.4 | 602 | 0.06 |

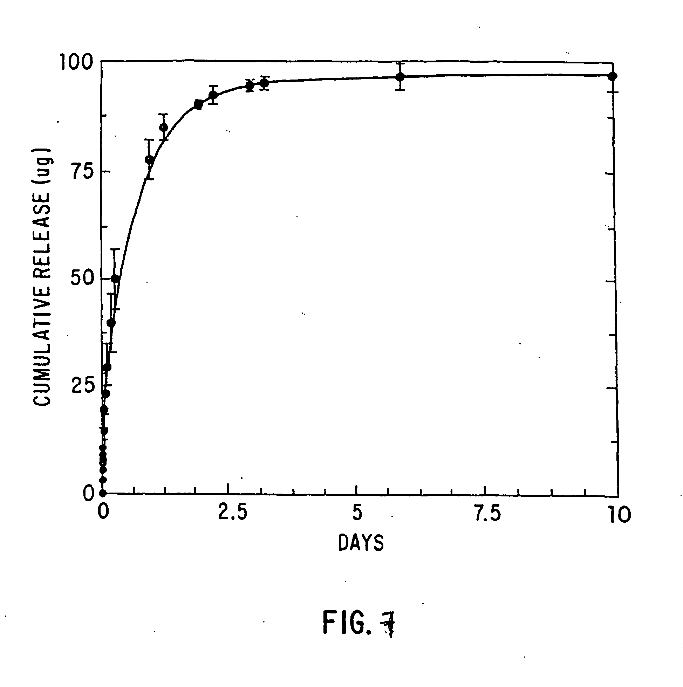

The remaining three stents are immersed in a solution of phosphate buffered saline solution having pH 7.4 at 37°C. Cumulative release as a function of time is presented in Fig. 7.

### Reference Example 8: Release kinetics of paclitaxel from polyurethane-based balloon catheter coating.

Nylon balloons are coated with polyurethane by dipping into a 9 wt% solution of CHRONOFLEX polyurethane in dimethylacetamide. The balloons are dried in a vacuum oven overnight at 50°C.

Each polyurethane coated balloon is loaded with paclitaxel either by dipping the coated balloon into a paclitaxel and ethanol solution or by dripping a known volume of a paclitaxel and ethanol solution onto the balloon surface.

In the first instance, a stock saturated solution of paclitaxel in ethanol is prepared. Then the polyurethane-coated balloon is inflated and submerged in the paclitaxel stock solution in a tube. The tube and balloon are well-sealed to prevent solvent evaporation. After remaining in the tube overnight, the ethanol is evaporated from the balloon over a suitable time period, such as about fifteen minutes. Five "dip-coated" balloons are prepared in this fashion.

In the second instance, a stock solution of paclitaxel having a concentration of 10 mg/ml prepared. Twenty ml of this paclitaxel stock solution are then pipetted onto an inflated polyurethane-coated balloon, providing a total mass of 200 mg of paclitaxel per balloon. Afterwards, ethanol is evaporated from the balloon over a suitable time period, such as about fifteen minutes. Five "drip-coated" balloons are prepared in this fashion.

Two drip-loaded balloons and two dip-loaded balloons are taken and the paclitaxel extracted in dichloromethane to determine total paclitaxel content. The paclitaxel content of the dip-coated balloons is found to be 1093 +/- 439 µg, while the drip-coated balloons are found to have 215 +/- 11 µg paclitaxel. For comparison, nylon balloons are coated with paclitaxel/polyurethane by dipping the balloons into a dispersion of 14.5 wt% BAYHYDROL polyurethane (made by Bayer) and 2.6 wt% paclitaxel in a mixture of 73.6 vol% N-methylpyrrolidinone and 26.4 vol% water. Balloons are dried in a vacuum oven overnight at 50°C. The dried coatings contain 15% paclitaxel by weight. Nine balloons are formed. Seven balloons are tested for paclitaxel loading yielding an average of 196 +/- 44 µg paclitaxel after extraction in dichloromethane.

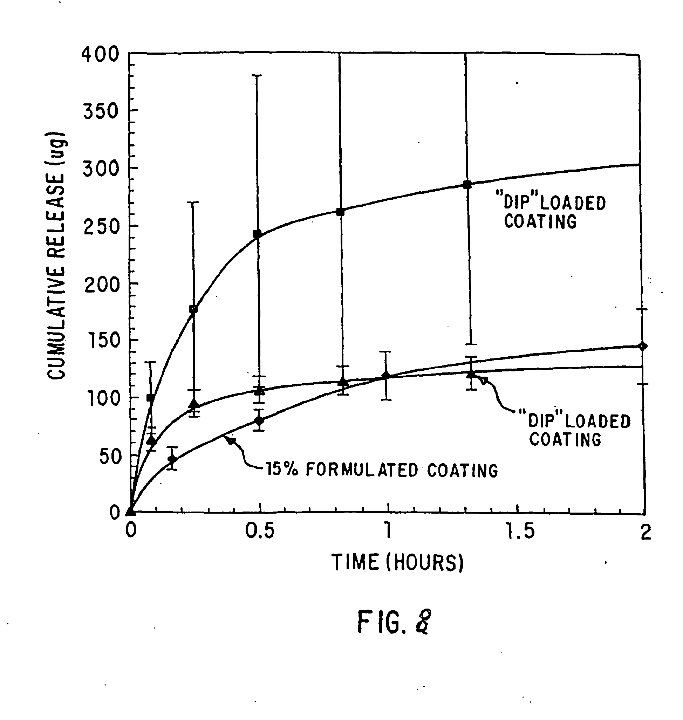

The remaining three drip-loaded balloons from above, the remaining three dip-loaded balloons from above, and the remaining two balloons with the 15% paclitaxel formulated coating are placed in a solution of phosphate buffered saline solution having pH 7.4 at 37°C, and cumulative paclitaxel release is measured as a function of time. The results of this study are presented in Fig. 8.

It is to be appreciated that the parameters described in the above examples are merely illustrative and that the present invention is not limited to such parameters. For example, in each of the examples provided, any suitable polymer may be used for the polymer coating, any suitable drying time periods and temperatures may be used, any suitable organic solvent may be used, any suitable method for applying the polymer coatings to the medical devices may be used, any suitable method for applying the drugs to the polymer coatings may be used, any suitable water-insoluble analogue of the disclosed drugs may be used, and any suitable drug loading concentrations may be used.

The present invention provides a previously unknown method and medical device for the localized delivery of substantially water-insoluble drugs. Those with skill in the art may recognize various modifications to the embodiments of the invention described and illustrated herein.

## Claims

1. A method comprising the steps of:
a) providing a polymer;
b) providing a medical device that is a balloon catheter adapted for insertion in a body;
c) coating at least a portion of the exterior surface of the medical device with the polymer to form a polymer coating, wherein the polymer is selected from the group consisting of polycarboxylic acids, cellulosic polymers, gelatin, polyvinylpyrrolidone, maleic anhydride polymers, polyamides, polyvinyl alcohols, polyethylene oxides, glycosaminoglycans, polysaccharides, polyesters, polyacrylamides, polyethers, acrylic latex dispersions, and mixtures and copolymers thereof;
d) applying a drug solution to the polymer coating after step c), said drug solution comprising at least one substantially water-insoluble drug dissolved in an organic solvent; and
e) drying said polymer coating such that substantially all of said solvent is evaporated.

2. The method of claim 1, wherein said polymer is polyacrylic acid.

3. The method of claim 1, wherein said at least one substantially insoluble drug is selected from the group consisting of dexamethasone, molsidomine, prednisolone, corticosterone, budesonide, estrogen, sulfasalazine, mesalamine, paclitaxel, cisplatin, vinblastine, vincristine, epothilones, endostatin, angiostatin, lidocaine, bupivacaine and ropivacaine.

4. The method of claim 1, wherein said organic solvent is selected from the group consisting of ethanol, isopropanol, chloroform, acetone, pentane, hexane, methylene chloride, and mixtures thereof.

5. The method of claim 4, wherein said organic solvent includes water.

6. The method of claim 1, wherein said step of applying a drug solution to said polymer includes the step of dipping said polymer into said drug solution.

7. The method of claim 1, wherein said balloon catheter comprises a shaft and an expandable portion mounted on said shaft, at least a portion of the exterior surface of the expandable portion being covered with said polymer coating.

8. The method of claim 1, wherein said step of coating comprises the step of applying multiple layers of said polymer to said medical device.

9. A medical device that is a balloon catheter for delivering substantially water-insoluble drugs to a desired location within a body, comprising:
a medical device adapted for insertion in a body; and
a polymer coating containing at least one substantially water-insoluble drug provided on at least a portion of said medical device, wherein said substantially water-insoluble drug has a water-solubility no greater than I part drug to 30 parts water, wherein said polymer is selected from the group consisting of polycarboxylic acids, cellulosic polymers, gelatin, polyvinylpyrrolidone, maleic anhydride polymers, polyamides, polyvinyl alcohols, polyethylene oxides, glycosaminoglycans, polysaccharides, polyesters, polyacrylamides, polyethers, acrylic latex dispersions, and mixtures and copolymers thereof

10. The medical device of claim 9, wherein said drug has a water solubility no greater than 1 part to 1,000 parts water.

11. The medical device of claim 10, wherein said balloon catheter comprises:
a shaft;
an expandable portion mounted on said shaft; and
a polymer coating on at least a portion of said expandable portion of said catheter, said polymer coating being impregnated with at least one substantially water-insoluble drug.

12. The medical device of claim 11, wherein said expandable portion includes an inflatable balloon.

13. The medical device of claim 12, further comprising a sheath member extendable over said expandable portion.

14. The medical device of claim 9, wherein said polymer is polyacrylic acid.

15. The medical device of claim 9, wherein said at least one substantially water-insoluble drug is selected from the group consisting of dexamethasone, molsidomine, prednisolone, corticosterone, budesonide, estrogen, sulfasalazine, mesalamine, paclitaxel, cisplatin, vinblastine, vincristine, epothilones, endostatin, angiostatin, lidocaine, bupivacaine and ropivacaine.

16. The medical device of claim 9, wherein said polymer coating is layered.

## Patentansprüche

1. Ein Verfahren beinhaltend die Schritte:
a) Bereitstellen eines Polymers;
b) Bereitstellen einer medizinischen Vorrichtung, die ein Ballonkatheter ist, angepasst zum Einführen in den Körper;
c) Beschichten zumindest eines Teils der äußeren Oberfläche der medizinischen Vorrichtung mit dem Polymer, um eine Polymerbeschichtung zu formen, wobei der Polymer ausgewählt ist aus der Gruppe bestehend aus Polycarboxylsäuren, Zellulosepolyemeren, Gelatine, Polyvinylpyrrolidone, Maleinsäureanhydridpolymere, Polyamide, Polyvinylalkohole, Polyethylenoxide, Glycosaminoglycane, Polysaccharide, Polyester, Polyacrylamide, Polyether, Acryl-Latexdispersionen, und Mischungen und Co-Polymere davon;
d) Anbringen einer Wirkstofflösung auf die Polymerbeschichtung nach Schritt c), wobei die Wirkstofflösung zumindest einen im Wesentlichen wasserunlöslichen Wirkstoff, gelöst in einem organischen Lösungsmittel, enthält; und
e) Trocknen der Polymerbeschichtung so, dass im Wesentlichen das gesamte Lösungsmittel verdunstet.

2. Das Verfahren nach Anspruch 1, wobei das Polymer Polyacrylsäure ist.

3. Das Verfahren nach Anspruch 1, wobei der zumindest im Wesentlichen unlösliche Wirkstoff ausgewählt ist aus der Gruppe bestehend aus Dexamethason, Molsidomin, Prednisolon, Corticosteron, Budesonid, Östrogen, Sulfasalazin, Mesalamin, Paclitaxel, Cisplatin, Vinblastin, Vincristin, Epothilone, Endostatin, Angiostatin, Lidocain, Bupivacain und Ropivacain.

4. Das Verfahren nach Anspruch 1, wobei das organische Lösungsmittel ausgewählt ist aus der Gruppe bestehend aus Ethanol, Isopropanol, Chloroform, Aceton, Pentan, Hexan, Methylenchlorid, und Mischungen daraus.

5. Das Verfahren nach Anspruch 4, wobei das organisches Lösungsmittel Wasser enthält.

6. Das Verfahren nach Anspruch 1, wobei der Schritt des Anbringens einer Wirkstofflösung zum Polymer einen Schritt des Eintauchens des Polymers in die Wirkstofflösung beinhaltet.

7. Das Verfahren nach Anspruch 1, wobei der Ballonkatheter einen Schaft und einen auf den Schaft angebrachten expandierbaren Teil umfasst, wobei zumindest ein Teil der äußeren Oberfläche des expandierbaren Teils mit der Polymerbeschichtung beschichtet ist.

8. Das Verfahren nach Anspruch 1, wobei der Schritt des Beschichtens den Schritt des Anbringens mehrerer Schichten des Polymers an das medizinische Gerät umfasst.

9. Eine medizinischer Vorrichtung, die ein Ballonkatheter zum Ausbringen im Wesentlich wasserunlöslichen Wirkstoffe an einen gewünschten Ort im Körper ist, umfassend:
eine medizinische Vorrichtung, angepasst zum Einführen in den Körper; und
eine Polymerbeschichtung, bestehend aus zumindest einem im Wesentlichen wasserunlöslichen Wirkstoff, bereitgestellt auf zumindest einem Teil der medizinischen Vorrichtung,
wobei der im Wesentlichen wasserunlösliche Arzneistoff eine Wasserlöslichkeit von nicht mehr als 1 Teil Arzneistoff auf 30 Teile Wasser hat, und
wobei das besagte Polymer ausgewählt ist aus der Gruppe bestehend aus Polycarboxylsäuren, Zellulosepolyemeren, Gelatine, Polyvinylpyrrolidone, Maleinsäureanhydridpolymere, Polyamide, Polyvinylalkohole, Polyethylenoxide, Glycosaminoglycane, Polysaccharide, Polyester, Polyacrylamide, Polyether, Acryl-Latexdispersionen, und Mischungen und Co-Polymere davon.

10. Die medizinische Vorrichtung nach Anspruch 9, wobei der Wirkstoff eine Wasserlöslichkeit nicht größer als 1 Teil in 1000 Teilen Wasser hat.

11. Die medizinische Vorrichtung nach Anspruch 10, wobei der Ballonkatheter umfasst:
einen Schaft;
einen expandierbaren Teil, befestigt auf dem Schaft; und
eine Polymerbeschichtung auf zumindest einem Teil des expandierbaren Teils des Katheters, wobei die Polymerbeschichtung mit zumindest einem im Wesentlichen wasserunlöslichen Wirkstoff imprägniert ist.

12. Die medizinische Vorrichtung nach Anspruch 11, wobei der expandierbare Teil einen aufblasbaren Ballon enthält.

13. Die medizinische Vorrichtung nach Anspruch 12, weiter umfassend eine Hülle, einschiebbar über den expandierbaren Teil.

14. Die medizinische Vorrichtung nach Anspruch 9, wobei das Polymer Polyacrylsäure ist.

15. Die medizinische Vorrichtung nach Anspruch 9, wobei der zumindest eine im Wesentlichen wasserunlösliche Wirkstoff ausgewählt ist aus der Gruppe bestehend aus Dexamethason, Molsidomin, Prednisolon, Corticosteron, Budesonid, Östrogen, Sulfasalazin, Mesalamin, Paclitaxel, Cisplatin, Vinblastin, Vincristin, Epothilone, Endostatin, Angiostatin, Lidocain, Bupivacain und Ropivacain.

16. Die medizinische Vorrichtung nach Anspruch 9, wobei die Polymerbeschichtung geschichtet ist.

## Revendications

1. Procédé comprenant les étapes consistant à :
a) fournir un polymère ;
b) fournir un dispositif médical consistant en un cathéter-ballon apte pour l'insertion dans un corps ;
c) revêtir au moins une portion de la surface extérieure du dispositif médical avec le polymère pour former un revêtement polymère, dans lequel le polymère est sélectionné parmi le groupe composé des acides polycarboxyliques, des polymères cellulosiques, de la gélatine, du polyvinylpyrrolidone, des polymères d'anhydride maléique, des polyamides, des alcools polyvinyliques, des oxydes de polyéthylène, des glycosaminoglycanes, des polysaccharides, des polyesters, des polyacrylamides, des polyéthers, des dispersions de polyuréthane, des dispersions de latex acrylique, et de leurs mélanges et de leurs copolymères ;
d) appliquer une solution médicamenteuse au revêtement polymère après l'étape c), ladite solution médicamenteuse comportant au moins un médicament sensiblement insoluble dans l'eau et dissout dans un solvant organique ; et
e) sécher ledit revêtement polymère de sorte que sensiblement la totalité du solvant soit évaporée.

2. Procédé selon la revendication 1, dans lequel ledit polymère est de l'acide polyacrylique.

3. Procédé selon la revendication 1, dans lequel au moins un médicament sensiblement insoluble est sélectionné parmi le groupe composé de la dexamétha¬sone, de la molsidomine, de la prednisolone, de la corticostérone, du budésonide, de l'oestrogène, de la sulfasalazine, de la mésalamine, du paclitaxel, du cisplatine, de la vinblastine, de la vincristine, des épothilones, de l'endostatine, de l'angiostatine, de la lidocaïne, de la bupivacaïne et de la ropivacaïne.

4. Procédé selon la revendication 1, dans lequel ledit solvant organique est sélectionné parmi le groupe composé de l'éthanol, de l'isopropanol, du chloroforme, de l'acétone, du pentane, de l'hexane, du chlorure de méthylène, et de leurs mélanges.

5. Procédé selon la revendication 4, dans lequel ledit solvant organique comporte de l'eau.

6. Procédé selon la revendication 1, dans lequel ladite étape d'application d'une solution médicamenteuse sur ledit polymère comporte l'étape consistant à plonger ledit polymère dans ladite solution médicamenteuse.

7. Procédé selon la revendication 1, dans lequel ledit cathéter-ballon comporte une tige et une portion extensible montée sur ladite tige, au moins une portion de la surface extérieure de la portion extensible étant recouverte dudit revêtement polymère.

8. Procédé selon la revendication 1, dans lequel ladite étape de revêtement comporte l'étape d'application de couches multiples dudit polymère sur ledit dispositif médical.

9. Dispositif médical consistant en un cathéter ballon pour délivrer des médicaments sensiblement insolubles dans l'eau à un emplacement désiré à l'intérieur d'un corps, comportant :
un dispositif médical adapté pour son insertion dans un corps ; et
un revêtement polymère contenant au moins un médicament sensiblement insoluble dans l'eau fournie sur au moins une portion dudit dispositif médical,
dans lequel le médicament pratiquement insoluble dans l'eau a une solubilité dans l'eau ne dépasse pas 1 partie du médicament à 30 parties d'eau, et
dans lequel ledit polymère est sélectionné parmi le group composé des acides polycarboxyliques, des polymères cellulosiques, de la gélatine, du polyvinylpyrrolidone, des polymères d'anhydride maléique, des polyamides, des alcools polyvinyliques, des oxydes de polyéthylène, des glycosaminoglycanes, des polysaccharides, des polyesters, des polyacrylamides, des polyéthers, des dispersions de polyuréthane, des dispersions de latex acrylique, et de leurs mélanges et de leurs copolymères.

10. Dispositif médical selon la revendication 9, dans lequel ledit médicament possède une solubilité dans l'eau ne dépassant 1 partie pour 1000 parties d'eau.

11. Dispositif médical selon la revendication 10, dans lequel ledit cathéter ballon comporte :
une tige ;
une portion extensible montée sur ladite tige ; et
un revêtement polymère sur au moins une portion de ladite portion extensible dudit cathéter, ledit revêtement polymère étant imprégné d'au moins un médicament sensiblement non soluble dans l'eau.

12. Dispositif médical selon la revendication 11, dans lequel ladite portion extensible comprend un ballon gonflable.

13. Dispositif médical selon la revendication 12, comportant en outre un élément de gaine extensible sur ladite portion extensible.

14. Dispositif médical selon la revendication 9, dans lequel ledit polymère est un acide polyacrilique.

15. Dispositif médical selon la revendication 9, dans lequel ledit au moins un médicament sensiblement insoluble dans l'eau est sélectionné parmi le groupe composé de la dexaméthasone, de la molsidomine, de la prednisolone, de la corticostérone, du budésonide, de l'oestrogène, de la sulfasalazine, de la mésalamine, du paclitaxel, du cisplatine, de la vinblastine, de la vincristine, des épothilones, de l'endostatine, de l'angiostatine, de la lidocaïne, de la bupivacaïne et de la ropivacaïne.

16. Dispositif médical selon la revendication 9, dans lequel ledit revêtement polymère est déposé en couches.
